# EUROPEAN PATENT SPECIFICATION

(11) **EP 1 743 042 B1**
(45) Date of publication and mention of the grant of the patent: **08.05.2013**
(21) Application number: 05729506.5
(22) Date of filing: 30.03.2005
(51) Int. Cl.: C12R 1/225, A23L 1/03, A23L 1/30, A23C 9/123, A23K 1/00, A61K 35/74, C12N 1/20

(54) **LACTIC ACID BACTERIA STRAINS EXHIBITING PROBIOTIC PROPERTIES AND COMPOSITIONS COMPRISING THE SAME**
PROBIOTISCHE EIGENSCHAFTEN ZEIGENDE MILCHSÄUREBAKTERIENSTÄMME UND DIESE UMFASSENDE ZUSAMMENSETZUNGEN
SOUCHES DE BACTERIES LACTIQUES AYANT DES PROPRIETES PROBIOTIQUES ET COMPOSITIONS COMPRENANT CES SOUCHES

(30) Priority: 31.03.2004 IT RM20040166
(43) Date of publication of application: 17.01.2007
(60) Divisional application: 10151690.4
(73) Proprietor: Synbiotec S.r.l., 62032 Camerino (MC) (IT)
(72) Inventor: Cresci, Alberto, 62032 Camerino MC (IT); Orpianesi, Carla, 62025 Fiuminata MC (IT); Silvi, Stefania, 62022 Castelraimondo MC (IT); Verdenelli, Maria Christina, 62010 Treia MC (IT)
(74) Representative: Predazzi, Valentina
(86) International application number: PCT/EP2005/051450
(87) International publication number: WO 2005/095656

(56) References cited:
- EP-A- 1 034 787
- OUWEHAND A C ET AL: "The mucus binding of Bifidobacterium lactis Bb12 is enhanced in the presence of Lactobacillus GG and Lact. delbrueckii subsp. bulgaricus." LETTERS IN APPLIED MICROBIOLOGY, vol. 30, no. 1, January 2000 (2000-01), pages 10-13, XP002336301 ISSN: 0266-8254
- HALLER D ET AL: "Metabolic and Functional Properties of Lactic Acid Bacteria in the Gastro-intestinal Ecosystem: A comparative in vitro Studybetween Bacteria of Intestinal and Fermented Food Origin" SYSTEMATIC AND APPLIED MICROBIOLOGY, vol. 24, no. 2, 2001, pages 218-226, XP004957293 ISSN: 0723-2020
- SILVI S ET AL: "EU project Crownalife: Functional foods, gut microflora and healthy ageing. Isolation and identification of Lactobacillus and Bifidobacterium strains from faecal samples of elderly subjects for a possible probiotic use in functional foods." JOURNAL OF FOOD ENGINEERING, vol. 56, no. 2-3, February 2003 (2003-02), pages 195-200, XP002336302 ISSN: 0260-8774 cited in the application
- MADSEN K ET AL: "Probiotic bacteria enhance murine and human intestinal epithelial barrier function." GASTROENTEROLOGY, vol. 121, no. 3, September 2001 (2001-09), pages 580-591, XP002336303 ISSN: 0016-5085
- LEE Y K ET AL: "Quantitative approach in the study of adhesion of lactic acid bacteria to intestinal cells and their competition with enterobacteria." APPLIED AND ENVIRONMENTAL MICROBIOLOGY, vol. 66, no. 9, September 2000 (2000-09), pages 3692-3697, XP002336304 ISSN: 0099-2240
- LEE Y-K ET AL: "Displacement of bacterial pathogens from mucus and Caco-2 cell surface by lactobacilli." JOURNAL OF MEDICAL MICROBIOLOGY, vol. 52, no. Pt 10, October 2003 (2003-10), pages 925-930, XP002336305 ISSN: 0022-2615
- OUWEHAND A C ET AL: "Assessment of adhesion properties of novel probiotic strains to human intestinal mucus." INTERNATIONAL JOURNAL OF FOOD MICROBIOLOGY. 28 FEB 2001, vol. 64, no. 1-2, 28 February 2001 (2001-02-28), pages 119-126, XP002336306 ISSN: 0168-1605
- GOPAL P K ET AL: "In vitro adherence properties of Lactobacillus rhamnosus DR20 and Bifidobacterium lactis DR10 strains and their antagonistic activity against an enterotoxigenic Escherichia coli." INTERNATIONAL JOURNAL OF FOOD MICROBIOLOGY. 5 AUG 2001, vol. 67, no. 3, 5 August 2001 (2001-08-05), pages 207-216, XP002336307 ISSN: 0168-1605
- VINDEROLA C G ET AL: "Interactions among lactic acid starter and probiotic bacteria used for fermented dairy products." JOURNAL OF DAIRY SCIENCE, vol. 85, no. 4, April 2002 (2002-04), pages 721-729, XP002336308 ISSN: 0022-0302
- FERNANDEZ M F ET AL: "Probiotic properties of human lactobacilli strains to be used in the gastrointestinal tract" JOURNAL OF APPLIED MICROBIOLOGY, vol. 94, no. 3, 2003, pages 449-455, XP002283214 ISSN: 1364-5072
- TUOMOLA E M ET AL: "Adhesion of some probiotic and dairy Lactobacillus strains to Caco-2 cell cultures." INTERNATIONAL JOURNAL OF FOOD MICROBIOLOGY, vol. 41, no. 1, 5 May 1998 (1998-05-05), pages 45-51, XP002336309 ISSN: 0168-1605 cited in the application
- BERNET M-F ET AL: "Adhesion of human bifidobacterial strains to cultured human intestinal epithelial cells and inhibition of enteropathogen-cell interactions" APPLIED AND ENVIRONMENTAL MICROBIOLOGY, vol. 59, no. 12, December 1993 (1993-12), pages 4121-4128, XP000857900 ISSN: 0099-2240
- GUSILS C ET AL: "Lactobacilli isolated from chicken intestines: potential use as probiotics." JOURNAL OF FOOD PROTECTION. MAR 1999, vol. 62, no. 3, March 1999 (1999-03), pages 252-256, XP008049839 ISSN: 0362-028X
- MORELLI L: "Invitro selection of probiotic Lactobacilli: a critical appraisal" CURRENT ISSUES IN INTESTINAL MICROBIOLOGY, vol. 1, no. 2, 2000, pages 59-67, XP001057348 ISSN: 1466-531X
- SAARELA M ET AL: "Probiotic bacteria: safety, functional and technological properties" JOURNAL OF BIOTECHNOLOGY, vol. 84, no. 3, 28 December 2000 (2000-12-28), pages 197-215, XP004314235 ISSN: 0168-1656
- DUNNE C ET AL: "In vitro selection criteria for probiotic bacteria of human origin: correlation with in vivo findings." THE AMERICAN JOURNAL OF CLINICAL NUTRITION, vol. 73, no. 2 Suppl, February 2001 (2001-02), pages 386S-392S, XP002336317 ISSN: 0002-9165
- DE VECCHI E ET AL: "Nature and modes of action of probiotics in intestinal infections." ANTIBIOTIQUES, vol. 5, no. 2, 2003, pages 107-113, XP008049894 ISSN: 1294-5501
- KARIMI O ET AL: "Probiotics: Isolated bacteria strain or mixtures of different strains? Two different approaches in the use of probiotics as therapeutics." DRUGS OF TODAY, vol. 39, no. 8, August 2003 (2003-08), pages 565-597, XP008049826 ISSN: 0025-7656
- KAUR I P ET AL: "Probiotics: Potential pharmaceutical applications" EUROPEAN JOURNAL OF PHARMACEUTICAL SCIENCES, vol. 15, no. 1, February 2002 (2002-02), pages 1-9, XP002232457 ISSN: 0928-0987
- ROLFE R D: "The role of probiotic cultures in the control of gastrointestinal health" JOURNAL OF NUTRITION, vol. 130, no. 2, SUPPL, 2000, pages 396S-402S, XP000978812 ISSN: 0022-3166

## Description

The present invention relates to the selection and the use of bacterial strains suitable for the preparation of foods and food supplements for the improvement or restoration of the intestinal microflora (gut microflora).

### STATE OF THE ART

It is known that several bacterial strains, e.g., lactic acid bacterial (LAB) strains among which those belonging to the genera *Lactobacillus* and *Bifidobacterium,* are normal components of the human and animal intestinal microflora.

Intestinal microflora comprises several bacterial species (human intestinal microflora, e.g., is represented by over 500 bacterial species) and significantly contributes to the maintenance of a good health status of the host. Moreover, it is known that deficiencies or damages to the intestinal microflora, both in humans and in several animals, are often correlated to an increase of illnesses, like, e.g., gastro-intestinal tract infections, irritable bowel disease, ulcerative colitides, alimentary allergies, antibiotic-induced diarrhoeas, cardiovascular disease and some kinds of tumour.

The beneficial features of several bacteria, like, e.g., the lactobacilli and the bifidobacteria, have been recognised in their role of normal components of the intestinal microflora, as well as in that of organisms administered as probiotics.

In particular, Ouwehand et al, in LETTERS IN APPLIED MICROBIOLOGY vol. 30, no.1 January 2000, pages 10-13, describe properties of Bifidobacterium lactis Bb12, Haller et al in SYSTEMATIC AND APPLIED MICROBIOLOGY vol. 24 no. 2, 2001 pages 218 226 describe metabolic and functional properties of Lactic Acid Bacteria in the human gastrointestinal system, and Madsen et al in GASTROENTEROLOGY vol. 121, no. 3 September 2001 pages 580-591 describe effects of probiotic bacteria on the murine and human intestinal epithelial barrier function.

Strains of these organisms are commonly used both for humans and animals, as therapeutical products, probiotics or added to food products because of their activities in the improvement of health.

The usefulness of these microorganisms, which may be administered by means of pharmaceutical compositions or through the assumption of probiotic foods comprising them, lies, e.g., in their ability to colonize the intestinal mucosa by adhering to its cells, replacing any pathogen species towards which they enact a competitive behaviour in the colonization. Hence, clearly the ability of the administered strains to adhere to the mucosa plays a key role in the effectiveness thereof, as it increases the probabilities that strains exhibiting high adhesion ability may supersede or prevent the adhesion of pathogens by a competitive action.

Moreover, it has been found that the intestinal microflora in the elderly undergoes modifications with respect to that of younger adult individuals or of youths. These modifications, like, e.g., the lesser presence of some genera such as the bifidobacteria or of some particular species, like, e.g., several species of lactobacilli, seem to be directly correlated to the higher susceptibility of elderly individuals to the above-indicated clinical forms (S.Silvi et al. Journal of Food Engineering Vol. 56, pp. 195-200 2003).

Microflora composition in the elderly further confirms the fact that there are situations, also physiological ones, in which the intestinal microflora is in a state compromised with respect to the optimal one. Hence, object of the present disclosure is to isolate novel probiotic strains exhibiting features such as to offer an optimal colonization, hence capable of giving medium-long term beneficial effects in the restoration of the intestinal microflora.

### SUMMARY OF THE INVENTION

The present invention relates to lactic acid bacterial strains as recited in claim 1, a pharmaceutical or veterinary composition as recited in claim 4, a process for the preparation of a pharmaceutical or veterinary composition as recited in claim 5, a probiotic food or food supplement as recited in claim 6, a process for the preparation of a probiotic food or food supplement as recited in claim 8, and a use as recited in claim 9. Additional embodiments of the invention are recited in the remaining claims.

In order to isolate and provide novel LAB bacterial strains, in the present disclosure known selection criteria have been considered in conjunction with innovative selection criteria. The known criteria considered are: resistance to acids, required so that the bacteria survive passing into the stomach; resistance to bile salts, it also necessary for the survival of the bacteria inside the digestive tract. However, these properties by themselves do not ensure the attainment of the desired effect. On the contrary, decisive is the ability of the bacterial strains to adhere to the intestinal mucosa, a property that has been considered as an additional selection criterion. As already indicated in the foregoing, an effective adhesion entails not only a likewise effective colonization of the intestinal mucosa, but also a strong competition toward any pathogenic strains. Hence, the greater is the adhesion ability the greater will be the effectiveness of the selected strain at replacing pathogens or preventing their colonization. On the basis of this criterion, bacterial strains characterized by a high adhesiveness, i.e. having adhesion values comparable to or higher than the maximum values known for each LAB species were selected.

However, besides the above-indicated parameters, an additional and previously neglected novel selection criterion was considered, allowing to identify strains particularly effective in the colonization of the intestinal mucosa. It is known that several bacterial strains can inhibit the growth of, or be inhibited in their growth by, other strains when co-inoculated therewith in a culture medium. Hence, the Authors of the present invention have considered the co-adhesion ability of strains identified in accordance with the preceding criteria as an additional selection criterion. In fact, it is not deemed sufficient that each of the selected strains have very high values of adhesivity (or adhesion) to the intestinal mucosa; yet, it is deemed essential that each of the selected strains be also capable of keeping said property in the presence of another strain selected in the same manner, so that the total adhesion values for pairs or groups of selected strains be at least equal to the sum of the adhesion values measured individually for each strain. This result is attained when each strain in the co-inoculation will have unaltered or even increased adhesion, whereas in the case of an adhesion decrease with respect to one or more strains, this will have to be balanced by an adhesion increase by other strains.

The application of said parameter, in addition to the ones described hereto, allows to further refine the selection and identify those strains having a very high probability of adhesion also in the presence of other strains, co-inoculated or naturally present in the intestine.

This property offers a two-fold advantage: the in vivo co-inoculation of two or more selected bacteria will lead to a very high general adhesion spectrum. However, even the mere inoculation of a single strain will have higher adhesion probabilities, not only due to its intrinsic high effectiveness, but also of its adhesion ability in the presence of other strains naturally belonging to the intestinal microflora of the host. In fact, the latter strains could compete with the administered strains, inhibiting them in their colonization and thwarting their action.

In the light of the foregoing, a process for the selection of bacterial strains suitable for restoring human or animal intestinal microflora is disclosed, comprising steps in which bacterial strains that have been isolated from human or animal faeces and cultured are preselected for their tolerance to gastric acids and bile salts, and subjected to additional steps of:
a) verifying *in vitro* the adhesion values of each strain to intestinal epithelium cells and selecting the strains exhibiting high adhesion, i.e., adhesion values comparable to or higher than the maximum values known for each corresponding species;
b) identifying, among these strains, single strains that, in a co-adhesion test with an additional strain selected by the same process, yield total adhesion values not lower than the sum of the adhesion values detected for each of the strains assayed individually;
c) isolating the strains, individually, in pair or in groups as identified in b).

Object of the invention are also single strains, pairs or groups of strains selected by said process, pharmaceutical or veterinary compositions or food supplements or probiotic foods comprising them, the processes for their preparation and the use of such strains individually, in pair or in group as active principles in probiotic foods.

The strains selected in accordance with the method of the disclosure, though suitable for the restoration of intestinal flora under any circumstance, will be particularly effective in those situations in which such a colonization is more difficult, like, e.g., in the elderly, in which it is proven that many bacterial species have a lesser survival and colonization ability.

### DETAILED DESCRIPTION OF THE FIGURES

Fig. 1 reports the growth curves, under conditions of anaerobiosis and of aerobiosis, of the bacterial strains *Lactobacillus rhamnosus* IMC 501 (Fig. 1a) and *Lactobacillus paracasei* IMC 502 (Fig. 1b); the rhombus-shaped symbols indicate the growth in anerobiosis, the square-shaped ones the growth in aerobiosis.
Figure 2 reports the viability of *Lactobacillus rhamnosus* IMC 501 (Fig. 2a) and *Lactobacillus paracasei* IMC 502 (Fig. 2b) strains during fermentation (20.5 hours at 37°C) and refrigerated storage (21 days at 4°C) in high-quality milk (AQ), whole milk (I) and semi-skim milk (PS).

### DETAILED DESCRIPTION OF THE INVENTION

Faecal sampling and isolation of the various bacterial strains may be carried out as taught in Silvi et al, "Journal of Food Engineering" 56 (2003) PP 195-200, or by any other suitable method known to a person skilled in the art. The initial selection media will be selected according to the bacterial genus that is desirable to isolate.

LAB bacteria can be isolated from human or animal faeces. Moreover, depending on the origin it is possible to carry out the selection on specific subgroups of subjects, like, e.g., persons suffering from a specific disorder, elderly people, subjects on specific diets related, e.g., to the environment in which they live, or animal families like canides, frelines, bovines, swine, ovines, equines, caprines, or other animal species.

In an embodiment of particular interest, the LAB strains are collected from elderly individuals, e.g., of an age ranging from 60 to 90 years. Anyway, it is advantageous that said subjects be healthy and not having been on antibiotic treatment at least in the four weeks prior to the faecal sampling.

The acid resistance assay may be carried out by any way known to a person skilled in the art, as long an acidity comparable to the gastric one of the host from which the strains have been isolated is taken into account. In human beings, e.g., gastric pH value ranges from about 1.5 and 4.5, in a 2-hour period, and depends on the ingestion time and the kind of gastric content. Of course, also the temperature at which the test is carried out should mimic a temperature comparable to body temperature.

A suitable assay for strains isolated from human faecal samples foresees the treatment of a known amount of cells of a fresh bacterial cuLture with citrate buffer at a pH of about 3 for about 5 hours at about 37°C, the centrifuging and the washing of the cells with a sterile physiological solution until elimination of the citrate buffer, the evaluation of cell survival with bacterial count techniques on a suitable medium. The survival rate will be given by the ratio between the number of cells survived to the treatment and the number of cells present prior to the acid treatment.

The bile salt tolerance assay may be carried out according to any known method. Said method should reproduce the physiological conditions; therefore, it would be advisable to verify the resistance to different concentrations of suitable bile salts at temperatures comparable to body temperatures. Said assay may be carried out by seeding a known amount of bacterial cells on a suitable medium containing bile salts at different concentrations, like, e.g.: Bile salt (purified and standardized bile extract, Oxoid) at the concentrations of 0.1%, 0.3%, 0.5%; or Bile salt No. 3 (purified deconjugated bile salts, Oxoid) at the concentrations of 0.05%, 0.1%, 0.2%. Cells are incubated at about 37°C in anaerobiosis for 24-48 hours, thereafter verifying the bacterial growth at the different concentrations and onto the different substrates. Survival to the treatment can be further confirmed by the subsequent growth, in a suitable culture medium, of the bacterial cells that have been exposed to bile salts.

For the adhesion assay, all known tests allowing to assess the adhesion values of each strain could be used. Thus, the adhesions of novel strains belonging to a determined species could be compared to those of known strains of the same species.

E.g., adhesion tests may be carried out by the processes described by Tuomola (née Lehto), et al.: Adhesion of some probiotic and dairy Lactobacillus strains to Caco-2 cell cultures - "International Journal of Food Microbiology" - 1998; 41:45-51 or by Blum, S. et al.: Adhesion of selected Lactobacillus strains to enterocyte-like Caco2 cells in vitro: a critical evaluation of reliability of in vitro adhesion assays. "4th Workshop, Demonstration of Nutritional Functionality of Probiotic Foods, Rovaniemi, Finland", February 25-28, 2000.

The method for assessing bacterial adhesion may be, e.g., carried out by using a cell line of human origin in a culture, as an *in vitro* model for intestinal epithelium. For instance, the known cell line HT-29, isolated from human colon adenocarcinoma, providing an advantageous cell model as it expresses *in vitro* a morphological and functional differentiation and exhibits the features of mature enterocytes, including polarization, a functional brush border and apical intestinal hydrolases.

Then, bacterial cell adhesion may be evaluated after having incubated, for 2 hours at about 37°C, a known-concentration bacterial suspension with a layer of confluenced HT-29 cells. After incubation, a bacterial count is carried out, e.g., with the method of serial dilutions and plate spreading, both of the supernatant and the cell layer. From the two bacterial counts there are obtained the number of non-adhering cells and the number of cells adhering to the cell layer", respectively. Then, the adhesion rate can be calculated by comparing the number of adhering cells to the total of the cells of the bacterial suspension used.

This method allows to define the percent values of adhesion typical of each preselected strain belonging to a given species, and therefore to compare the values found with the maximum values known for the same species, or to identify the maximum values for each species whose adhesion data are not available. Hence, the method allows to easily select from a known bacterial species novel bacterial strains, which in accordance with the invention exhibit adhesion values comparable to or greater than the maximum values known for that species. E.g., the adhesion values described in literature for known species are reported in Table I, Tuomola (*née* Letho) et al. (supra).

The maximum adhesion values are: for the *L. casei* species (Fyos®, Nutricia), subsequently identified by API CHL (4.0) method as *L. rhamnosus* species, 14.4 ± 3.9%, for the *L. acidophilus* 1 genus (LCI ®, Nestlé) 12.6 ± 2.5%, and for the *L*. *casei* species (BIO® Danone) subsequently identified by API CHL (4.0) method as *L*. *paracasei* species, 4.8 ± 1.3%.

The term "high adhesion" used in the present application indicates adhesion values comparable to or higher than the maximum values known for each given species, where for "comparable" it is meant values differing from the maximum values of no more than 0.5 percentage points.

The co-adhesion assays can be carried out analogously to the adhesion ones, using however bacterial suspension mixtures at known concentrations of each strain. Mixtures of two or more cultures could be used. The adhesion rate could then be calculated with respect to the mixture, thereby obtaining a total adhesion value, or with respect to each bacterial strain, thereby allowing to know the total adhesion value and the adhesion value exhibited by each co-incubated strain.

Therefore, to the ends of the invention there will be selected those strains that, following co-adhesion assays, yield a total adhesion equal to at least the sum of the adhesion values of each strain considered individually.

Advantageously, the co-adhesion test on groups of bacterial strains may be carried out by adding one strain at a time, starting from pairs of strains for which the satisfactory co-adhesion has already been verified. Thus, there could be selected whole bacterial groups whose co-adhesion value is equal at least to the sum of the single adhesion values.

Hence, by virtue of the assessment of the co-adhesion parameter, the process herein disclosed allows to identify and select not merely single strains exhibiting a high adhesion ability, but above all specific combinations of bacteria (pairs or groups) particularly suitable to the restoration of the intestinal bacterial microflora.

Evidently, both the selection of the single strains and the selection of plural strains having the desired features is extremely advantageous, as these strains, being particularly effective in the restoration of the intestinal microflora, will be as effective in their competitive action against pathogenic bacteria.

An additional property that may be evaluated for the selection of bacterial strains, obtained in pairs or groups at step c) of the process, is the presence or the absence of synergy in the growth in a culture medium. Thus, there could be identified all those strains exhibiting, in addition to the selected features, also a synergistic growth, advantageously allowing an obvious yield increase in the bacteria production step itself.

The process herein disclosed may comprise an additional step (d) apt to assay the tolerance to oxygen of each selected strain and to select the tolerant strains. Of course, the oxygen tolerance test can be carried out at any moment of the selection process, although it is clearly more systematic to carry out said assay only on the strains identified in step c) of the claimed process. Tolerance to oxygen can be verified in any one way known to a person skilled in the art. Such a verification may be carried out, e.g., by subjecting each strain of interest to a growth under aerobic conditions in a suitable culture medium for a period of about 24 hours, transferring the cells into a fresh medium, verifying their growth under anaerobic conditions and selecting the oxygen-tolerant strains.

The advantage of such a selection lies in the fact that the strains exhibiting tolerance to oxygen, and therefore ability to grow under both aerobic and anaerobic conditions, are more easily processable during the growth stage. Moreover, their resistance to oxygen causes the products comprising them, be those pharmaceutical compositions, veterinary compositions or probiotic foods, to require no specific storage and packaging apt to keep the bacteria under anaerobic conditions. Hence, from an end-product production and storage standpoint, the selection of oxygen-resistant bacteria is particularly advantageous.

Another property evaluated as additional selection criterion, step (e), is the ability of the strains, preselected individually, in pair or in group, to grow in milk without addition of further substances promoting their growth. Of course, such a selection step may be carried out at any one stage of the process, though it is more practical, as for step d), to verify it on the strains already pre-selected for co-adhesion.

The advantage of such a selection is evident for the preparation of probiotic foods or food supplements comprising milk, or milk-based, or milk derivative-based. In this case, the culture medium for the growth of the selected strains may be the milk itself, with no need of further purification treatments for the bacteria prior to their integration into the probiotic food comprising it, or with no need to add to said products one or more nutrients that might change food flavour or not be suitable for alimentary use.

Particularly suitable strains in accordance with the present invention are strains belonging to the genus *Lactobacillus* or *Bifidobacterium.* The pairs or groups of strains according to the invention could comprise strains belonging to one or both of the genera. In particular, the single strains, pairs or groups could belong to all species of lactobacilli and bifidobacteria or comprise, when in pair or in group, one or more species of lactobacilli and/or one or more species of bifidobacteria. Some examples of suitable species are *Bifidobacterium adolescentis, Bifidobacterium bifidum, Bifidobacterium longum, Lactobacillus acidophilus, Lactobacillus brevis, Lactobacillus buchneri, Lactobacillus cellobiosus, Lactobacillus delbuckii* spp. *bulgaricus, Lactobacillus fermentum, Lactobacillus paracasei* spp. *paracasei, Lactobacillus plantarum, Lactobacillus rhamnosus, Lactobacillus salivarius, Lactobacillus curvatus.*

In an embodiment according to the invention, the bacterial strains belong to the species *Lactobacillus rhamnosus* and/or *Lactobacillus paracasei.*

Moreover, the selection criteria could comprise one or more of the following selection parameters.

In a further embodiment, the strains of the invention exhibit resistance to antibiotics. This makes them particularly suitable as probiotic bacteria, to be administered above all concomitantly to antibiotic therapies.

Therefore, these strains could be selected also on the basis of an evidence of resistance to antibiotics.

Furthermore, strains according to the invention could exhibit inhibition activity on potentially pathogenic microorganisms (e.g., *Escherichia coli, Staphylococcus aureus, Candida albicans,* etc.). Of course, this makes them all the more interesting as probiotics. Therefore, also the parameter of the inhibition of potentially pathogenic microorganisms could constitute a selection parameter.

Lastly, strains according to the invention could exhibit a high conservation of the bacterial viability when inoculated in milk and stored at about 4°C. In an embodiment, the strains will exhibit a substantially unchanged bacterial viability even after 21 days of storage at about 4°C. This property could also constitute a selection parameter.

Advantageously, said strains could also not influence the pH of the milk in which they are stored, not fermenting therein without starter strains and not acidifying it. Also the verification of this property could be a selection parameter.

Strains of the invention are those deposited on December 08, 2003 at the DSMZ, Deutsche Sammlung Von Mikroorganismen Und Zellkulturen, Germany, in accordance with Budapest Treaty, and having deposit numbers DSM 16104 and DSM 16105. In the present description these strains are characterized as *Lactobacillus rhamnosus* IMC 501 DSM 16104 and *Lactobacillus paracasei* IMC 502 DSM 16105, respectively.

The pair of such strains or each group comprising such strains are embodiments of the invention.

In addition to the deposit number, said strains are also characterized by the following:
- Tolerance to acids (pH about 3);
- Tolerance to bile salts;
- ability to grow in aerobiosis as well as in anaerobiosis;
- ability to grow in milk without addition of further substances apt to promote the growth thereof;
- synergistic effect on the growth when co-inoculated;
- adhesion values:
   *Lactobacillus rhamnosus* IMC 501 DSM 16104 14.9% ± 3.2 and *Lactobacillus paracasei* IMC 502 DSM 16105 4.7% ± 1.5;
- unchanged adhesion values in the co-adhesion assay comprising both strains.

The bacteriological features of the two strains are as follows:

### Lactobacillus rhamnosus IMC 501 DSM 16104

*Morphological characteristics:* Gram-positive nonsporing rod-shaped microorganism, chain formation common. Colonies on Agar media appear spherical, with entire margins, whitish on MRS and yellowish on BHI (Brain Heart Infusion) .

*Cultural characteristic:* optimal growth temperature is of 34-39°C. Growth on a liquid medium occurs with the formation of a smooth and homogeneous sediment. Not forming pellicles.

*Sugar fermentation:* test carried out by API 50 CHL enzymatic kit (bioMérieux). The strain has been found positive to: ribose, galactose, D-glucose, D-fructose, D-mannose, L-sorbose, ramnose, mannitol, sorbitol, α-methyl-D-glucoside, N-acetyl-glucosamine, amygdalin, arbutin, salicin, cellobiose, maltose, lactose, sucrose, trehalose, melezitose, β-gentiobiose, D-turanose, D-tagatose, whereas it has been found negative to: glycerol, erythrol, D-arabinose, L-arabinose, D-xylose, L-xylose, adonitol, β-methyl-xyloside, dulcitol, inositol, α-methyl-D-mannoside, melibiose, inulin, D-raffinose, starch, glycogen, xylitol, D-lyxose, D-fucose, L-fucose, D-arabitol, L-arabitol, gluconate, 2-keto-gluconate, 5-keto-gluconate.

*Physiological characteristics:* the strain was found capable of hydrolyzing esculin.

### Lactobacillus paracasei IMC 502 DSM 16105

*Morphological characteristics:* Gram-positive microorganism, rod-shaped, often occurring in a 4-unit or chain arrangement. Colonies on Agar medium appear spherical, with entire margins. On MRS the colonies are whitish and shiny, whereas on BHI they exhibit a slightly raised central portion having transparent edges.

*Cultural characteristics:* the optimal growth temperature is of 34-39°C. Growth on a liquid medium occurs with the formation of a smooth and homogeneous sediment. Not forming pellicles.

*Sugar fermentation:* The strain has been found positive to: ribose, galactose, D-glucose, D-fructose, D-mannose, mannitol, sorbitol, N-acetyl-glucosamine, arbutin, salicin, cellobiose, maltose, sucrose, trehalose, inulin, β-gentiobiose, D-turanose, D-tagatose, whereas it has been found negative to: glycerol, erythrol, D-arabinose, L-arabinose, D-xylose, L-xylose, adonitol, β-methyl-xyloside, L-sorbose, ramnose, dulcitol, inositol, α-methyl-D-mannoside, α-methyl-D-glucoside, amygdalin, lactose, melibiose, melezitose, D-raffinose, starch, glycogen, xylitol, D-xylose, D-fucose, L-fucose, D-arabitol, L-arabitol, gluconate, 2-keto-gluconate, 5-keto-gluconate.

*Physiological characteristics:* the strain was found capable of hydrolyzing esculin.

Both the IMC 501 and the IMC 502 strains exhibit resistance to several antibiotics, like vancomycin, colistin sulfate, oxolinic acid and penicillin G, and keep the same bacterial viability when stored in milk at about 4°C for at least 21 days. Moreover, the strains do not acidify the milk in which they are stored.

According to the invention, the single, pairs or groups of strains selected may also be in lyophilized form. Lyophilization may be carried out according to any one method known to a person skilled in the art. Likewise, the bacteria of the invention could be dehydrated, like, e.g., described in Canadian Pat. CA2263954.

The strains of the invention, selected individually, in pairs or in group, are useful as medicaments. Two kinds of pharmaceutical or veterinary compositions may be made: the first composition will comprise at least one of the strains obtainable by the selection process herein disclosed, the second one will comprise at least one of the pairs or one of the group of strains obtainable thereby. Along with the selected bacteria, in the composition there will be one or more pharmaceutically acceptable excipients, and, optionally, additional active principles.

Besides at least one of the strains obtainable by the selection process herein disclosed, the composition could also comprise additional individually selected strains. Alternatively, the composition could comprise one or more pairs or one or more groups selected according to the invention. However, such a composition could also be limited to a single novel bacterial strain obtainable by the process herein disclosed in conjunction with suitable excipients and optional additional active principles and/or aromatizers and/or sweeteners. Such additional active principles could also be known LAB strains.

In the latter case, the composition should comprise at least one pair or group of strains obtainable by the described process, yet it could also comprise additional strains individually selected with the same process or other active principles, which could be known LAB strains, along with pharmaceutically acceptable excipients and additives.

Said pharmaceutical compositions could comprise the bacteria in lyophilized form or in non-lyophilized form; therefore, it could be in powder, tablets, capsules, liquids, dense or semi-solid liquids. When these compositions comprise bacteria in lyophilized form, the latter will generally be in a lyophilized and inactivated form, in an amount ranging from about 5 to about 10 billions per dose. Suitable excipients could be: culture medium brought to neutrality and lyophilized, calcium carbonate, silicic acid, talc, magnesium stearate, anhydrous lactose, jelly. Optionally, there could be added also an aroma apt to make the pharmaceutical preparation palatable and a sweetener (sucrose, fructose, etc.).

Excipients suitable for preparations in liquid form could instead be, e.g., purified water, and the total concentration of the bacteria could be of from 1 to 10 billions per dose, preferably about 5 billions.

Examples of pharmaceutical formulations and preparation thereof are known to the art; suitable to the ends of the present invention may be the processes indicated in the published Pat. App. US2004/0057943, as well as other processes known to a person skilled in the art. The bacteria could be in suspension, lyophilized or inactivated, as long as they are not killed. Therefore, the preparation of the compositions of the invention could be carried out by lyophilizing bacterial cultures, mixing cultures lyophilized or in suspension with water or additional suitable excipients and optionally with the addition of further active principles. In an alternative embodiment of the invention, the selected bacterial st rains are comprised in probiotic foods or flood supplements.

These may be in liquid, semi-liquid, solid or powder form and should comprise at least one of the bacterial strains or at least one pair or group of bacterial strains of the invention, suitable food ingredients and, optionally, preservatives, acidifiers, antioxidants, additional probiotic bacterial strains, food supplements and/or excipients.

Therefore, the foods could comprise a single bacterial strain, or one pair or group of bacterial strains, yet could comprise additional strains, pairs or groups in any mutual combination. Moreover, also known LAB strains could be added to the novel strains of the invention.

Probiotic foods of particular interest could be dairy products or dairy-based products, and could also be or comprise dehydrated milk.

The foods of the invention may be prepared in any way known to a person skilled in the art by mixing at least one of the bacterial strains, or at least one pair or group of the bacterial strains of the invention, be them fresh, lyophilized, inactivated or dehydrated (as long as not killed), to suitable food ingredients and, optionally, to preservatives, acidifiers, antioxidants, additional probiotic bacterial strains, food supplements and/or excipients.

For the preparation of dehydrated probiotic milk or of a food comprising it, milk may be fermented with LAB bacteria of the invention, optionally with additional known LAB bacteria. Then, sodium glutamate and ascorbic acid are added and the fermented milk is dried by atomization. The solid thus obtained may then be processed to form tablets, sweets or other kinds of food.

Probiotic yoghurts comprising the bacteria of the invention may be prepared by techniques known to a person skilled in the art, like, e.g., those described in Intl. Pat. App. WO-A-03090546 or in US Pat. No. 6,599,504 or in EU Pat. App. No. EP-A-1243180.

The amount of bacteria that should be present in the foods is indicated in said reference, and anyhow known to a person skilled in the art. Generally, probiotic foods comprise from 1 to 10 million bacteria per gram. Said bacteria may be grown directly in the food, in the starting material, or added at any stage of food preparation. The addition may be carried out by adding the medium (e.g., milk) in which the bacteria have grown, or after purification of the latter therefrom.

The disclosure also provides methods for the traceability of the bacteria of the invention by sequencing with standard techniques or suitable commercially available 16S rDNA kits.

In fact, 16S rDNA comprises regions highly conserved in bacteria interposed to hypervariable regions. These hypervariable regions comprise sequences characterizing phylogenetic groups and even species. The taxonomic information contained in 16S rDNA, exhibiting strain-specific features, constitutes the foundation of the molecular analytic methods, it being suitable for a quick identification of the bacterial strains.

Hence, from the sequence at issue there may be constructed, with standard methodologies or programs, primers allowing the traceability of the desired strains both in the functional products and for research purposes.

Hereinafter, examples will be provided, given purely by way of illustration and not limitative of the scope of the claims.

### EXAMPLES:

### EXAMPLE 1

### Isolation and selection of the bacterial strains

Faeces taken from elderly subjects, of both sexes and ranging in age from 60 to 90 years were collected in sterile containers and put in a plastic watertight bag under anaerobic conditions obtained by the use of "AnaeroGen" sachets (OXOID-UNIPATH Ltd., Basingstoke, Hampshire, England). The samples were stored at +4° C for a maximum time period of 24 h and within that time transferred, in an anaerobic cabinet (Concept 400, Ruskinn Technology Limited, Leeds, West Yorkshire, UK) to a laboratory.

For isolation, media selective for the *Lactobacillus* genus were used: LAMVAB agar (*Lactobacillus* Anaerobic MRS with Vancomycin and Bromocresol Green) and MRS (de Man, Rogosa, Sharpe) agar (Oxoid). The inoculated selective media were incubated in anaerobiosis at 37°C for 72 h. 54 *Lactobacillus spp.* bacterial strains were isolated.

Identification of said strains was carried out by API 50 CHL enzymatic method (bioMérieux, Marcy l'Etoile, France).

Said strains were subjected to assays of tolerance to gastric acids, bile salts and oxygen. *Lactobacillus rhamnosus* IMC 501 and *Lactobacillus paracasei* IMC 502 survived all tolerance assays.

### EXAMPLE 2

### Inoculum growth and storage methods

*Lactoba cillus rhamnosus* IMC 501 and *Lactobacillus paracasei* IMC 502 are cultivated on MRS (de Man, Rogosa and Sharpe) medium. Growth occurs under aerobic and anaerobic conditions at 37°C after a 24-48 h incubation.

The strains can be stored for a short period at 4°C, for a month on agar slants, whereas for longer periods the cells can be transferred into a storage system at - 80°C using the Microbank system (PRO-LAB DIAGNOSTIC, NESTON, UK) or lyophilized after having added 20% skim milk and stored at room temperature.

The composition of the MRS medium is as follows:
**MRS** (de Man, Rogosa, Sharpe) (amounts expressed in g/l) :

| | |
|---|---|
| Peptone | 10.0 |
| Lab-Lemco | 8.0 |
| Yeast extract | 4.0 |
| Glucose | 20.0 |
| Tween 80 | 1 ml |
| Potassium phosphate monoacid | 2.0 |
| Sodium acetate 3H₂O | 5.0 |
| Triammonium citrate | 2.0 |
| Magnesium sulphate 7H₂O | 0.2 |
| Manganese sulphate 4H₂O | 0.05 |
| pH 6,2 ± 0, 2 | |

### EXAMPLE 3

### Assay of bacterial adhesion on intestinal epithelium

The bacterial ability to adhere to epithelial cells is considered as one of the selection criteria for the probiotic strains.

Owing to the difficulty of studying the bacterial adhesion ability *in vivo,* the bacterial adhesion assay of the two strains onto the intestinal epithelium was studied by using a cell line of human origin in a culture, as an *in vitro* model for intestinal epithelium.

The cell line used is HT-29, isolated from human colon adenocarcinoma, one of the cell lines more often used in order to evaluate the adhesion ability of probiotic strains. The advantage of this cell model is that it expresses *in vitro* a morphological and functional differentiation and exhibits the features of mature enterocytes, including polarization, a functional brush border and apical intestinal hydrolases.

Bacterial cell adhesion was evaluated after having incubated for 2 hours at 37°C a known-concentration bacterial suspension with a layer of confluenced HT-29 cells. After incubation, a bacterial count was carried out with the method of serial dilutions and plate spreading, both of the supernatant and the cell layer.

From the two bacterial counts there have been obtained the number of non-adhering cells and the number of cells adhering to the cell layer, respectively.

The adhesion rate was calculated by comparing the number of adhering cells to the total of the cells of the bacterial suspension used.

*Lactobacillus rhamnosus* IMC 501 exhibits an adhesion rate of 14.9% ± 3.2 and *Lactobacillus paracasei* IMC 502 of 4.7% ± 1.5, which, compared to the known values of commercial strains belonging to the same species (Table 1), are among the highest.

**Table 1**

| Adhesion rates of commercially available probiotic bacterial strains | | |
|---|---|---|
| **Bacterial strains** | **API CHL (4.0) Identification** | **Adhesion (%), (mean+S.D.)** |
| *L. casei* (Fyos®, Nutricia) | *L. rhamnosus* | 14.4±3.9 |
| *L. acidophilus* 1 (LC1®, Nestlé) | *L. acidophilus* | 12.16±2.5 |
| *Lactobacillus* GG ATCC 53103 | *L. rhamnosus* | 9.7±3.3 |
| *L. casei* (BIO®, Danone) | *L. paracasei* | 4.8±1.3 |
| *L. casei* Immunitas (Actimel®, Danone) | *L. paracasei* | 4.4±1.1 |
| *L. casei* Shirota (Yakult®, Yacult) | *L paracasei* | 3.2±0.52 |
| *L. casei* var- *rhamnosus* (Lactophilus®, Laboratoires Lyocentre) | *L. rhamnosus* | 2.6±0.69 |

### EXAMPLE 4

### Growth curves under conditions of anaerobiosis and of aerobiosis:

By using the spectrophotometric method, the bacterial growth curve of strains *Lactobacillus rhamnosus* IMC 501 and *Lactobacillus paracasei* IMC 502 in an MRS liquid medium was determined, keeping the optimal conditions of pH and temperature and monitoring the growth under conditions of anaerobiosis as well as of aerobiosis for a time of nine hours.

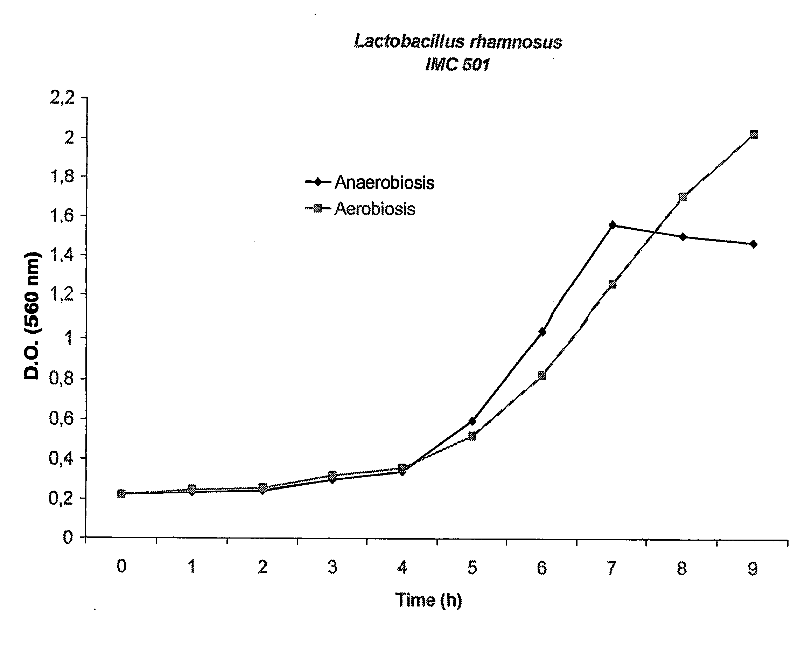

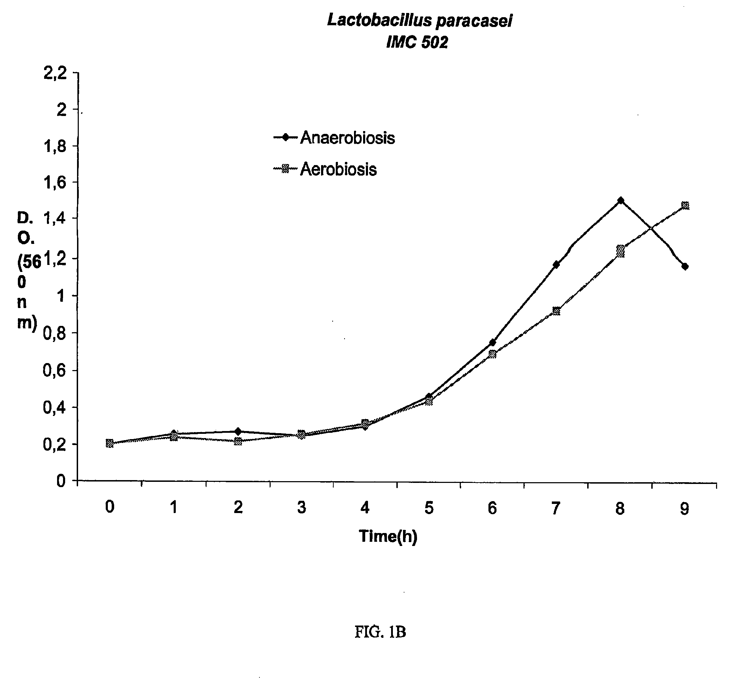

Figure 1 reports the growth curves of the two bacterial strains.

*Lactobacillus rhamnosus* IMC 501 exhibits, in the exponential growth stage under conditions of anaerobiosis, higher D.O. values until the seventh hour; thereafter, the growth in aerobiosis has higher values.

IMC 502 *Lactobacillus paracasei* exhibits, in the exponential growth stage under conditions of anaerobiosis, higher D.O. values until the eighth hour; thereafter, the growth in aerobiosis is found to be better.

### Example 5

### a. Antibiotics sensitivity test

The antibiotics resistance profile of the two bacterial strains *Lactobacillus rhamnosus* IMC 501 and *Lactobacillus paracasei* IMC 502 was assayed by using the classic Agar diffusion method. A total of 11 antibiotic substances, i.e., ampicillin, erithromycin, rifampicin, neomycin, amoxicillin, colistin sulfate, oxolinic acid, penicillin G, polymyxin, chloramphenicol and vancomycin were assayed. Both strains were found resistant to vancomycin, colistin sulfate, oxolinic acid and penicillin G and sensitive to the other 7 antibiotics tested.

### b. Antimicrobial activity evaluation

The inhibiting effects of the two bacterial strains on potentially pathogenic microorganisms like *Escherichia coli, Staphylococcus aureus* and *Candida albicans* were studied by using the "deferred cross-streak" modified technique described in Fang et al.: Antagonism of lactic acid bacteria towards Staphylococcus aureus and Escherichia coli on agar plates and in milk - "Veterinary Research" - 1996; 27: 3-12. The inhibition activity of the two probiotic strains was evaluated on the basis of the inhibition zone diameter against common intestinal pathogens. The results are reported in Table 2.

**Table 2.**

| Degree of inhibition of Probiotic strains against potential intestinal pathogens. | | | |
|---|---|---|---|
| **Probiotic strains** | **E. coli** (ATCC 11775) | **S. aureus** (ATCC 25923) | **C**. **albicans** (ATCC 10291) |
| *Lactobacillus rhamnosus* IMC 501 | ++ | ++ | +++ |
| *Lactobacillus paracasei* IMC 502 | ++ | ++ | +++ |

| | | | |
|---|---|---|---|
| ++ inhibition zone > 2 x 2 cm +++ inhibition zone > 3 x 3 cm | | | |

As it evident from the abovereported results, both strains exhibited a good inhibition on the growth of the 3 strains tested, particularly enhanced towards *Candida albicans.*

### Example 6

### Technological properties of the Probiotic strains

By using pH measuring and viability evaluation, the ability of the two bacterial strains *Lactobacillus rhamnosus* IMC 501 and *Lactobacillus paracasei* IMC 502 to acidify milk was evaluated. For this test, three different kinds of milk were used: whole homogenized pasteurized fresh milk, homogenized semi-skim pasteurized fresh milk and high-quality fresh milk. The milks were inoculated with a suspension of the two bacterial strains and left fermenting for about 20.5 h at 37°C. The process was monitored by pH measuring. Then, the products were stored at about 4°C for 21 days, carrying out a count of bacterial viability on MRS agar after the inoculation, after the fermentation process and after 7, 14 and 21 days of storage.

None of the two strains was capable of acidifying the milk, therefore being particularly suitable for integrating fresh milk without varying its pH.

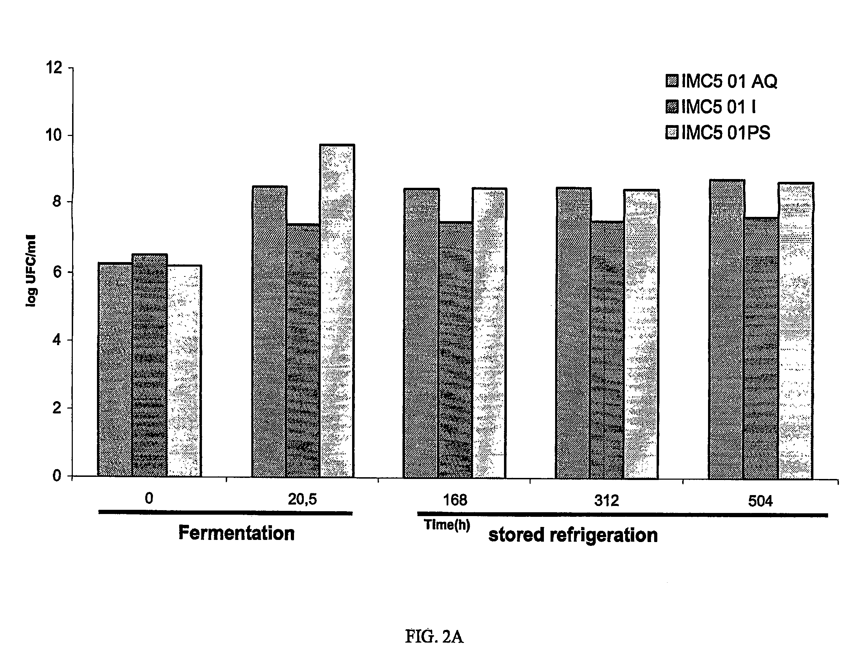

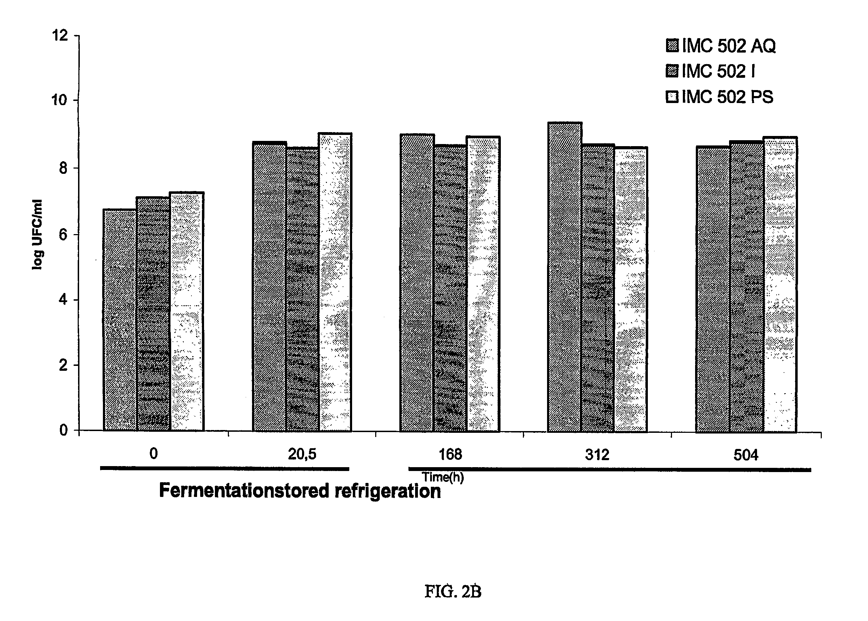

Both strains remained viable during the storage at 4°C up to 21 days after inoculation, and the pH value of the milk products was constant for the entire period (Fig. 2).

Moreover, in-milk survival of the probiotic strains of the invention during cold storage was evaluated. After inoculation, the sample was stored at about 4°C for 21 days. Strains viability was analyzed by count on MRS plates after inoculation and after 7, 14 and 21 days of storage. Both strains kept the bacterial count present in the inoculation up to 21 days of storage at 4°C.

### Example 7

### Probiotic strains sequencing

To determine the sequence of the 16S rDNA of the probiotic strains, a DNA extraction was carried out by using the Dnase Tissue kit (Qiagen); then, a PCR reaction was prepared, using primers P0 and P6 corresponding to positions 27f (forward) and 1495r (reverse) of *Escherichia coli* 16S rDNA. Upon determining the occurred amplification by electrophoresis, the PCR products were purified by Qiagen kit and then sequenced. The sequences thus obtained are used, with standard programs and systems, for the construction of species-specific primers, which may be used in the traceability of the desired probiotic strains, as well as in functional products and in the study on human beings.

## Claims

1. Lactic acid bacterial strains suitable for restoring human or animal intestinal microflora having *in vitro* adhesion values comparable to or higher than the maximum values known for each corresponding species and having a total co-adhesion value not lower than the sum of the adhesion values of each strain to intestinal epithelium cells detected for each of the strains assayed individually, **characterized in that** they are selected from the strains having deposit number DSM 16104 and DSM 16105.

2. The bacterial strains according to claim 1, in lyophilized form.

3. The bacterial strains according to any one of the claims 1 or 2, for use as medicament.

4. A pharmaceutical or veterinary composition comprising the bacterial strains according to claim 3 and pharmaceutically acceptable excipients and, optionally, additional bacterial strains and/or active principles.

5. A process for the preparation of a pharmaceutical or veterinary composition according to claim 4, **characterized by** mixing the bacterial strains according to claim 3 to one or more pharmaceutically acceptable excipients and, optionally, to additional bacterial strains and/or active principles.

6. A probiotic food or food supplement, in liquid, thickened liquid, semisolid, solid or powder form, comprising the bacterial strains according to claims 1 or 2, suitable food ingredients, and, optionally, preservatives, acidifiers, antioxidants, additional probiotic bacterial strains, food supplements and/or excipients.

7. The food or food supplement according to claim 6, **characterized in that** they are dairy product-based.

8. A process for the preparation of a probiotic food or food supplement according to claims 6 or 7, **characterized by** mixing the bacterial strains according to claims 1 or 2, to suitable food ingredients and, optionally, to preservatives, acidifiers, antioxidants, additional probiotic bacterial strains, food supplements, and/or excipients.

9. Use of the bacterial strains according to claims 1 or 2, for the preparation of probiotic foods.

## Patentansprüche

1. Milchsäurebakterienstämme geeignet zur Wiederherstellung menschlicher oder tierischer Darmmikroflora, welche in vitro-Adhäsionswerte haben, die vergleichbar mit oder höher sind als die maximalen Werte, die für jede entsprechende Spezies bekannt sind, und welche einen Gesamt-Koadhäsionswert haben, der nicht niedriger als die Summe der Adhäsionswerte jedes Stammes zu Darmepithelzellen ist, die für jeden der getesteten Stämme einzeln nachgewiesen wurden, **dadurch gekennzeichnet, dass** sie ausgewählt sind aus den Stämmen mit den Hinterlegungsnummern DSM 16104 und DSM 16105.

2. Bakterienstämme nach Anspruch 1 in lyophilisierter Form.

3. Bakterienstämme nach einem der Ansprüche 1 oder 2 zur Verwendung als Medikament.

4. Pharmazeutische oder veterinärmedizinische Zusammensetzung umfassend die Bakterienstämme nach Anspruch 3 und pharmazeutisch verträgliche Exzipienten und, gegebenenfalls, zusätzliche Bakterienstämme und/oder Wirkstoffe.

5. Verfahren zur Herstellung einer pharmazeutischen oder veterinärmedizinischen Zusammensetzung nach Anspruch 4, **gekennzeichnet durch** das Mischen der Bakterienstämme nach Anspruch 3 mit einem oder mehreren pharmazeutisch verträglichen Exzipienten und, gegebenenfalls, mit zusätzlichen Bakterienstämmen und/oder Wirkstoffen.

6. Probiotisches/r Nahrungsmittel oder Nahrungsmittelzusatz in flüssiger, verdickt flüssiger, halbfester, fester oder pulvriger Form, umfassend die Bakterienstämme nach Anspruch 1 oder 2, geeignete Nahrungsmittelinhaltsstoffe, und, gegebenenfalls, Konservierungsmittel, Säuerungsmittel, Antioxidantien, zusätzliche probiotische Bakterienstämme, Nahrungsmittelzusätze und/oder Exzipienten.

7. Nahrungsmittel oder der Nahrungsmittelzusatz nach Anspruch 6, **dadurch gekennzeichnet, dass** sie auf Milchprodukten basieren.

8. Verfahren zur Herstellung eines probiotischen Nahrungsmittels oder Nahrungsmittelzusatzes nach Anspruch 6 oder 7, **gekennzeichnet durch** Mischen der Bakterienstämme nach Anspruch 1 oder 2 mit geeigneten Nahrungsmittelinhaltsstoffen und, gegebenenfalls, Konservierungsmitteln, Säuerungsmitteln, Antioxidantien, zusätzlichen probiotischen Bakterienstämmen, Nahrungsmittelzusätzen und/oder Exzipienten.

9. Verwendung der Bakterienstämme nach Anspruch 1 oder 2 zur Herstellung von probiotischen Nahrungsmitteln.

## Revendications

1. Souches de bactéries lactiques appropriées pour restaurer la microflore intestinale chez un humain ou un animal, présentant des indices d'adhérence *in vitro* de valeurs comparables ou supérieures aux valeurs maximales connues pour chaque espèce correspondante et présentant un indice total de co-adhérence au moins égal à la somme des indices d'adhérence de chaque souche aux cellules de l'épithélium intestinal déterminés pour chacune des souches testées individuellement, **caractérisées en ce qu'**elles sont choisies parmi les souches dont les numéros de dépôt sont DSM 16104 et DSM 16105.

2. Souches de bactéries conformes à la revendication 1, sous forme lyophilisée.

3. Souches de bactéries conformes à la revendication 1 ou 2, pour utilisation en tant que médicament.

4. Composition pharmaceutique ou vétérinaire comprenant des souches de bactéries conformes à la revendication 3 et des excipients pharmacologiquement admissibles, et en option, des souches bactériennes et/ou des principes actifs supplémentaires.

5. Procédé de préparation d'une composition pharmaceutique ou vétérinaire conforme à la revendication 4, **caractérisé en ce que** l'on mélange des souches de bactéries conformes à la revendication 3 avec un ou plusieurs excipient(s) pharmacologiquement admissible(s), et en option, avec des souches bactériennes et/ou des principes actifs supplémentaires.

6. Aliment ou complément alimentaire probiotique, sous forme de liquide, de liquide épais, de semi-solide, de solide ou de poudre, comprenant des souches de bactéries conformes à la revendication 1 ou 2 et des ingrédients alimentaires appropriés, et en option, des conservateurs, des acidifiants, des anti-oxydants, des souches bactériennes probiotiques supplémentaires, des compléments alimentaires et/ou des excipients.

7. Aliment ou complément alimentaire conforme à la revendication 6, **caractérisé en ce qu'**il est à base de produit laitier.

8. Procédé de préparation d'un aliment ou complément alimentaire probiotique conforme à la revendication 6 ou 7, **caractérisé en ce que** l'on mélange des souches de bactéries conformes à la revendication 1 ou 2 avec des ingrédients alimentaires appropriés, et en option, avec des conservateurs, des acidifiants, des anti-oxydants, des souches bactériennes probiotiques supplémentaires, des compléments alimentaires et/ou des excipients.

9. Utilisation de souches de bactéries conformes à la revendication 1 ou 2 pour la préparation d'aliments probiotiques.
